# EUROPEAN PATENT SPECIFICATION

(11) **EP 1 998 205 B1**
(45) Date of publication and mention of the grant of the patent: **06.07.2011**
(21) Application number: 08008670.5
(22) Date of filing: 08.05.2008
(51) Int. Cl.: G02B 21/00, G02B 21/06, G02B 21/18, G01N 21/00

(54) **Observation apparatus**
Beobachtungsgerät
Appareil d'observation

(30) Priority: 29.05.2007 JP 2007142129; 26.06.2007 JP 2007167746
(43) Date of publication of application: 03.12.2008
(73) Proprietor: Olympus Corporation, Tokyo 151-0072 (JP)
(72) Inventor: Natori, Yasuaki, Tokyo 193-0816 (JP); Honda, Susumu, Hachioji-shi Tokyo 192-0046 (JP)
(74) Representative: von Hellfeld, Axel

(56) References cited:
- EP-A- 1 686 407
- WO-A-03/021241
- WO-A-2005/029152
- DE-A1-102005 008 619
- JP-A- 8 138 609
- US-A- 5 202 744
- US-A- 5 932 871
- US-A- 6 121 616

## Description

### BACKGROUND OF THE INVENTION

### 1. FIELD OF THE INVENTION

The present invention relates to observation apparatuses.

This application is based on Japanese Patent Application No. 2007-142129 and No. 2007-167746.

### 2. DESCRIPTION OF RELATED ART

A known in-vivo observation apparatus in the related art uses a high-sensitivity monochrome CCD camera to observe luminescence or fluorescence from a living organism which serves as a subject to be observed (for example, see the Publication of Japanese Patent No. 3786903).

In addition, a known in-vivo observation apparatus in the related art uses a color CCD camera to observe fluorescence having multiple wavelengths emitted from a fluorescent material or the like administered to a living organism which serves as a subject to be observed (for example, see Japanese Translation of PCT International Application, Publication No. 2006-514830).

Also known in the related art is an observation apparatus for observing a specimen dyed with a plurality of fluorescent dyes or a specimen in which a plurality of fluorescent proteins are expressed (for example, see Japanese Unexamined Patent Application, Publication No. 2004-177662).

With this observation apparatus, the specimen is irradiated with light having a plurality of wavelengths, and the light returning from the specimen is collected by an objective lens, is separated into each wavelength by a dichroic mirror before being imaged by an image-forming lens, and is acquired by a plurality of image-acquisition units.

With the high-sensitivity monochrome CCD camera disclosed in the Publication of Japanese Patent No. 3786903, it is possible to observe weak fluorescence from the living organism; however, there is a problem in that light cannot be detected in color. In contrast, with the color CCD camera disclosed in Japanese Translation of PCT International Application, Publication No. 2006-514830, it is possible to detect light from the living organism in color; however, there is a problem in that it cannot detect weak light or light in the near-infrared wavelength band.

In addition, the cameras used for these in-vivo observation apparatuses are usually optically aligned with the observation optical systems provided in the apparatuses. Accordingly, precise optical alignment is required each time the cameras are replaced. Or, sometimes optical alignment may not be possible or replacement of the cameras themselves may not be possible.

With the observation apparatus disclosed in Japanese Unexamined Patent Application, Publication No. 2004-177662, the return light from the specimen, collected by the objective lens, is divided at a point between the image-forming lens and the image-acquisition units. However, when a reduction optical system with a low magnification is needed, as with the living organism observation apparatus, it is difficult to ensure a wide space between the image-forming lens and the image-acquisition units, resulting in the inability to form a similar mechanism.

Document EP 1 686 407 A1 concerns a confocal laser scanning microscope having an exchangeable optical source and a detector connection unit. In one embodiment, the detector connection unit is realized as a screw mechanism for fixing two optical fibres with each other.

Document WO 03/021241 A1 concerns a fluorescence detection instrument having reflective transfer legs for color decimation. By means of stand-off supports, a plurality of clusters of the instrument can be vertically mounted on a rack.

Document DE 10 2005 008 619 A1 concerns a microscope having a receiving block comprising inlets into which detectors may be fixed.

Document US 5,202,744 concerns an electro-optical measurement instrument having an optical routing module for routing a primary light beam comprising visible light from an illuminator to a sample disposed on a sample stage and subsequently to a CCD camera and a binocular eyepiece.

The present invention has ueen conceived in light of the circumstances described above, and an object thereof is to provide an observation apparatus in which various kinds of observation can be precisely carried out using different types of image-acquisition unit and to enable low-magnification observation. Additionally, it is also an object to provide an observation apparatus capable of acquiring return light from a single specimen using a plurality of image-acquisition units.

### BRIEF SUMMARY OF THE INVENTION

To realize the objects described above, the present invention provides an observation apparatus having the features of claim 1.

According to the invention, by irradiating the specimen with the illumination light emitted from the illumination light source, the return light, such as fluorescence or reflected light, returning from the specimen is collected by the objective lens and imaged at the image-acquisition unit by the image-forming lens. Accordingly, an image of the specimen is acquired by the image-acquisition unit. In this case, because the image-acquisition unit is attached by the attaching-and-detaching mechanism in such a manner that it is capable of being attached and detached while being positionally aligned with the optical axis of the image-forming lens, it is possible to replace the image-acquisition unit with a different type, such as a monochrome CCD camera or a color CCD camera. Because the attaching-and-detaching mechanism attaches and detaches the image-acquisition unit in such a manner that it can be positionally aligned with the optical axis of the image-forming lens, even when replacing the image-acquisition unit with a different type, it is possible to acquire a clear image by optically aligning each image-acquisition unit.

In the invention, the attaching-and-detaching mechanism may be detachable together with the image-acquisition unit, and the attaching-and-detaching mechanism may have a position adjustment mechanism configured to positionally align the image-acquisition unit with the optical axis of the image-forming lens.

In this way, the attaching-and-detaching mechanism can be provided in each image-acquisition unit, and the part that is attached to/detached from the observation apparatus can be provided with a common design for all image-acquisition units. By operating the position adjustment mechanism, each of the image-acquisition units is positionally aligned with the optical axis of the image-forming lens; therefore, various types of image-acquisition units can be selectively attached, allowing various kinds of observation.

In the configuration of the invention, the position adjustment mechanism may move the image-acquisition unit in the direction of an optical axis of the image-forming lens and in the direction orthogonal to the optical axis.

In this way, the optical axis of the image-acquisition unit can be easily aligned with the optical axis of the image-forming lens, and also, an image-acquisition sensor surface of the image-acquisition unit can be precisely aligned with an image position of the image-forming lens.

In the configuration described above, the position adjustment mechanism may be capable of independently moving the image-acquisition unit in the direction of the optical-axis of the image-forming lens and in the direction orthogonal to the optical axis.

In this way, position adjustment mechanism can independently align the image-acquisition unit in the direction of the optical-axis of the image-forming lens and in the direction orthogonal to the optical-axis, thus allowing easy alignment.

### BRIEF DESCRIPTION OF THE SEVERAL VIEWS OF THE DRAWINGS

Fig. 1 is a diagram showing the overall configuration of an observation apparatus according to the embodiment of the present invention.
Fig. 2 is a longitudinal sectional view for explaining an attaching-and-detaching mechanism of the observation apparatus in Fig. 1.
Fig. 3 is a diagram for explaining the operation of replacing CCD cameras used for different kinds of observation with the observation apparatus shown in Fig. 1.
Fig. 4 is a diagram showing the overall configuration of an observation apparatus according to a non claimed example.
Fig. 5 is an elevational view for explaining a switching mechanism of the observation apparatus in Fig. 4.
Fig. 6A is a diagram for explaining the operation of switching CCD cameras used for different kinds of observation with the observation apparatus in Fig. 4.
Fig. 6B is a diagram for explaining the operation of replacing CCD cameras used for different kinds of observation with the observation apparatus in Fig. 4.
Fig. 7 is a diagram showing the overall configuration of an observation apparatus according to another non claimed example.
Fig. 8 is a diagram showing the overall configuration of a modification of the observation apparatus in Fig. 7.

### DETAILED DESCRIPTION OF THE INVENTION

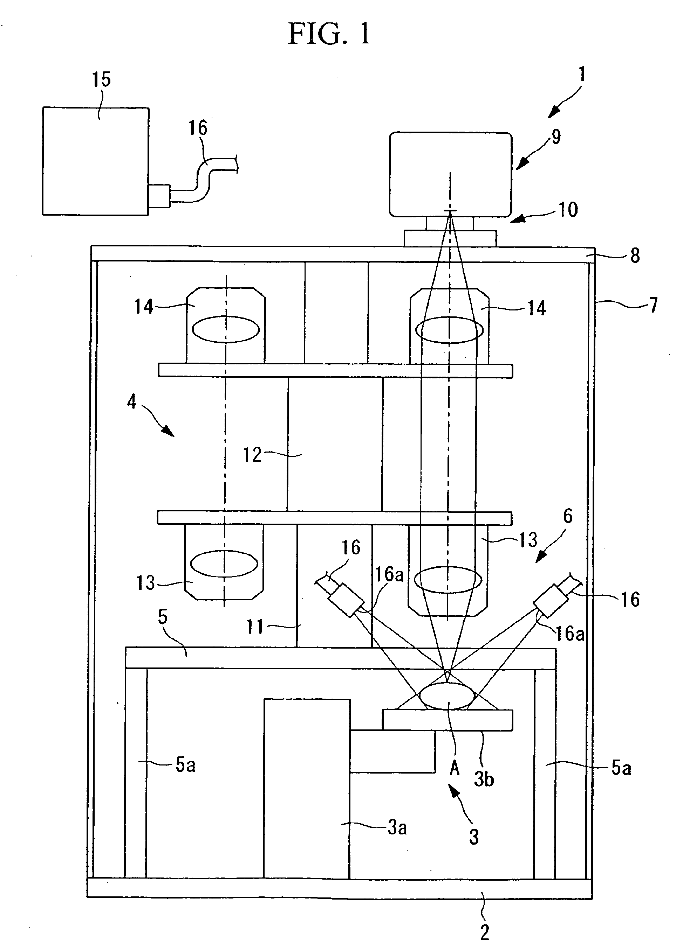

An observation apparatus 1 according to a first embodiment of the present invention will be described below with reference to Figs. 1 to 3.

The observation apparatus 1 according to this embodiment is suitable for observing a living organism, such as a small laboratory animal like a mouse, as a specimen A.

As shown in Fig. 1, the observation apparatus 1 according to this embodiment includes a stage 3 for mounting the specimen A, the stage 3 being disposed on a first base 2 that is horizontally disposed on an installation surface; an observation optical system 4 disposed above the stage 3; a second base 5 for mounting the observation optical system 4; an illumination apparatus 6 for irradiating the specimen A on the stage 3 with illumination light; a light-shielding member 7 for covering them; a CCD camera 9 that is attached to a top plate 8 of the light-shielding member 7; and an attaching-and-detaching mechanism 10 for attaching the CCD camera 9 to the top plate 8 in such a manner that it is capable of being attached and detached.

The stage 3 includes a Z stage 3a for moving the specimen A in the vertical direction, and an XY stage 3b for two-dimensionally moving the specimen A in the horizontal direction.

The second base 5 is disposed above the stage 3 with a space provided therebetween by a spacing member 5a and has a support stand 11 vertically extending therefrom. The support stand 11 has a turret 12 that is rotatable about the vertical axis.

The observation optical system 4 includes a plurality of pairs of objective lenses 13 and image-forming lenses 14 that are secured to the turret 12 and that have different magnifications. The high-magnification objective lenses 13 and image-forming lenses 14, and the low-magnification objective lenses 13 and image-forming lenses 14 are disposed so as to be aligned with the optical axis.

The illumination apparatus 6 includes an excitation light source 15 for emitting excitation light and an optical fiber 16 for guiding the excitation light emitted from the excitation light source 15. The excitation light source 15 is disposed outside the light-shielding member 7, and exit ends 16a of the optical fiber 16 are disposed close to the stage 3 inside the light-shielding member 7. In this way, the excitation light emitted from the excitation light source 15 can be guided by the optical fiber 16 so as to irradiate the specimen A mounted on the stage 3.

The light-shielding member 7 completely covers the stage 3, the second base 5, and the observation optical system 4 to block light so that external light does not get inside. In addition, the light-shielding member 7 blocks the excitation light emitted from the exit ends 16a of the optical fiber 16 to prevent the excitation light from leaking outside.

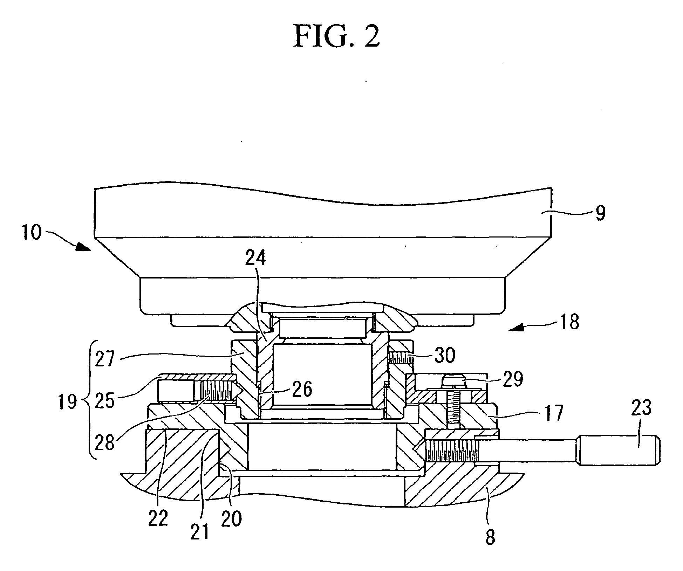

As shown in Fig. 2, the attaching-and-detaching mechanism 10 includes a removable mounting member 17 that is attached to the top plate 8 in such a manner that it is capable of being attached and detached; a movable mounting member 18, which is supported by the removable mounting member 17 in a manner allowing the movable mounting member 18 to move and to which the CCD camera 9 is attached; and a position adjustment mechanism 19 for positionally aligning the movable mounting member 18.

The removable mounting member 17, which includes a fitting portion 21 that fits in a fitting hole 20 provided in the top plate 8 and an abutting surface 22 that is brought in close contact with the upper surface of the top plate 8, performs positioning in a direction intersecting the optical axis by fitting the fitting portion 21 in the fitting hole 20 and performs positioning in a direction parallel to the optical axis by abutting the abutting surface 22 against the upper surface of the top plate 8. In addition, the removable mounting member 17 is secured to the top plate 8 by a set screw 23 by pressing the outer circumferential surface of the fitting portion 21 of the removable mounting member 17 inwards in the radial direction.

The movable mounting member 18 includes a camera mount 24 for mounting the CCD camera 9.

The position adjustment mechanism 19 includes an XY mount 25 which is attached to the removable mounting member 17 in such a manner that the XY mount 25 is movable in two horizontal directions; a θ mount 27, which is attached to the XY mount 25 in such a manner that the θ mount 27 is rotatable about the optical axis and which supports the camera mount 24 so as to move in the optical-axis direction by means of a lead screw 26 whose central axis is the optical axis; and securing screws 28, 29, and 30 for securing them at desired positions.

The operation of the observation apparatus 1 according to this embodiment, configured in this way, will be described below.

To carry out observation of the specimen A using the observation apparatus 1 according to this embodiment, first, the CCD camera 9 is attached to the camera mount 24 provided on the movable mounting member 18, and the optical axis of the CCD camera 9 is aligned by operating the position adjustment mechanism 19.

Next, a non claimed method for aligning the observation optical axis with the center of the sensor of the CCD camera 9 using the position adjustment mechanism 19 will be described.

First, in order to align the optical axis, it is necessary to dispose a target, serving as an alignment reference, at the center of the observation optical axis and in the focal plane of the objective lens 13. In order to do so, crosshairs (not shown in the drawing) serving as the target are drawn in advance at the center of the surface of the XY stage 3b of the observation apparatus 1.

In addition, the XY stage 3b and the Z stage 3a are motorized stages that can be controlled by a control computer (not shown in the drawing). During assembly and alignment performed in advance at the factory, the crosshairs are aligned so as to be positioned at the center of the observation optical axis and in the focal plane of the objective lens 13, and positional information about the crosshairs used for coordinate information is stored in the control computer as coordinate data using a coordinates function of the motorized stages. When aligning the CCD camera 9, it is possible for the XY stage 3b and the Z stage 3a to make the crosshairs reappear at the center of the observation optical axis and in the focal plane of the objective lens 13 by retrieving the coordinate data from the control computer.

By moving the crosshairs to the center of the observation optical axis and in the focal plane of the objective lens 13 using the control computer, the position of the sensor surface of the CCD camera 9 is aligned with the focal plane of the image-forming lens 14.

In order to carry out the above alignment, first, the camera mount 24 and the CCD camera 9 that is secured thereto are moved in the optical-axis direction by an amount based on the lead of the lead screw 26, by rotating the θ mount 27 about the optical axis while checking an image on a monitor acquired by the CCD camera 9. Thereafter, the camera mount 24 is secured to the θ mount 27 by the securing screw 30, and the θ mount 27 is secured to the XY mount 25 by the securing screw 28, while the crosshairs are clearly displayed on the monitor.

Next, the center position of the image-acquisition sensor surface of the CCD camera 9 is aligned with the center of the crosshairs by operating the XY mount 25. Specifically, while displaying a mark indicating the center position of the image-acquisition sensor surface on the monitor and while checking the crosshairs displayed on the monitor, the XY mount 25 is moved in directions orthogonal to the optical axis with respect to the removable mounting member 17 so as to be aligned with the mark. In this state, the XY mount 25 is secured to the removable mounting member 17 by the securing two screws 29 or more.

Accordingly, the optical axis of the CCD camera 9 can be aligned with the optical axis of the observation optical system 4, and the image-acquisition sensor surface can be aligned with the focal plane of the image-forming lens 14.

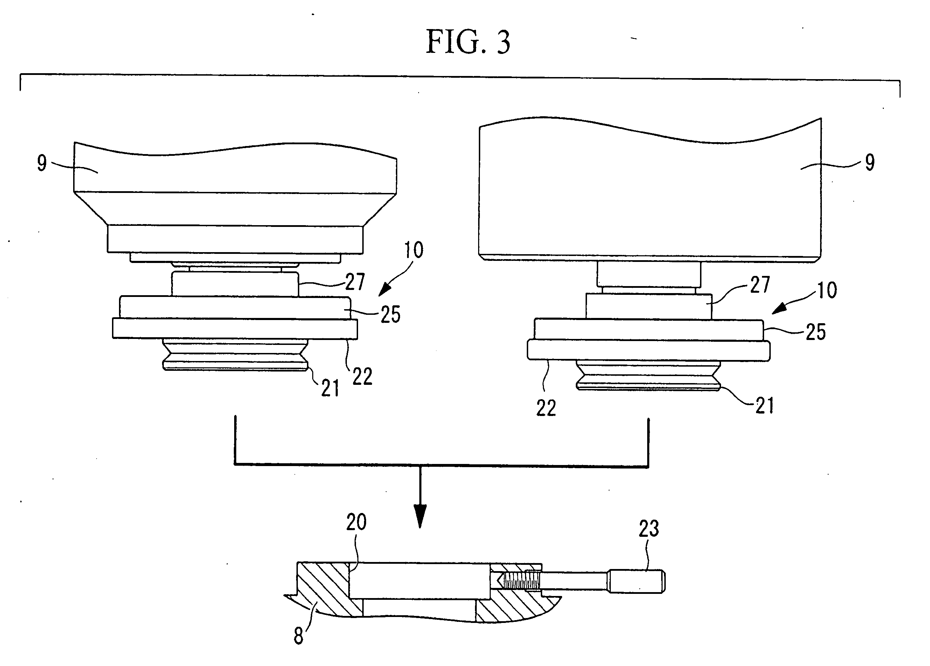

When observation is to be carried out using a different type of CCD camera 9, the CCD camera 9 that was previously used is detached together with the removable mounting member 17 by loosening the set screw 23, another type of CCD camera 9 having the same attaching-and-detaching mechanism 10, as shown in Fig. 3, is mounted, and the optical axis and focal position are aligned in a similar manner to that described above. In this way, even when performing a different kind of observation, it is possible to easily replace the CCD camera 9, for example, with a color CCD or a monochrome CCD suitable for the particular observation, thus avoiding the need for complicated positional alignment when replacing cameras. Accordingly, an advantage is afforded in that various kinds of observation can be easily carried out.

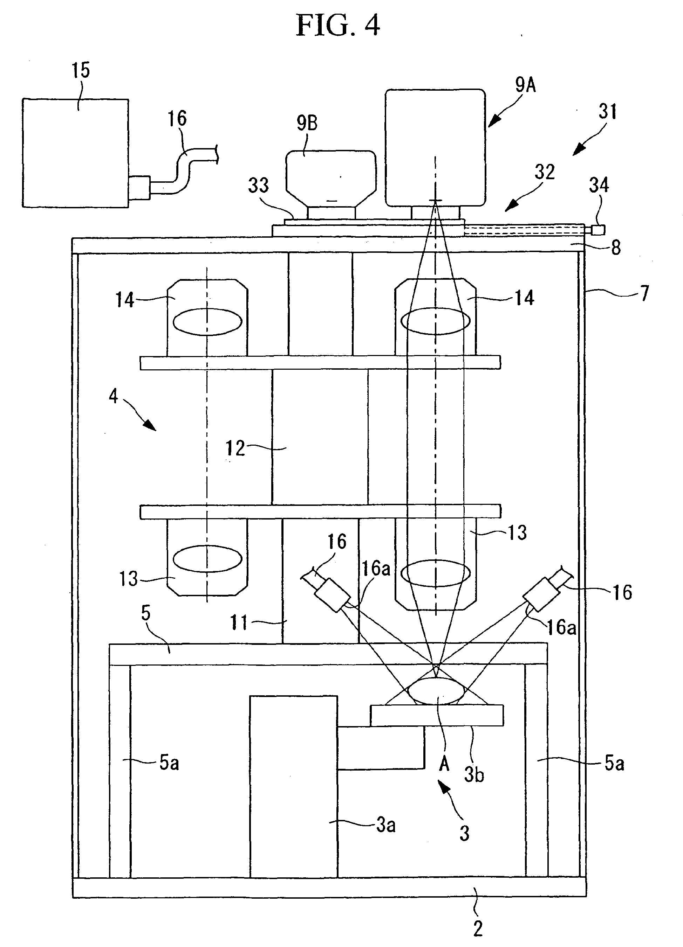

Next, an observation apparatus 31 according to a non claimed example will be described below with reference to Figs. 4 to 6B.

In the description of this example, parts having the same configuration as those of the observation apparatus 1 according to the embodiment described above are assigned the same reference numerals, and a description thereof will be omitted here.

The observation apparatus 31 according to this example differs from the observation apparatus 1 according to the embodiment in that it includes a switching mechanism 32 for switching among different types of CCD cameras 9A and 9B while being positionally aligned with the optical axis of the observation optical system 4.

As shown in Figs. 4 to 6B, the switching mechanism 32 includes a slider 33, which is provided on the top plate 8 in such a manner as to be linearly movable in the horizontal direction and to which two different types of CCD cameras 9A and 9B are attached. Reference numeral 34 in the drawing is a switching knob which is operated to move the slider 33.

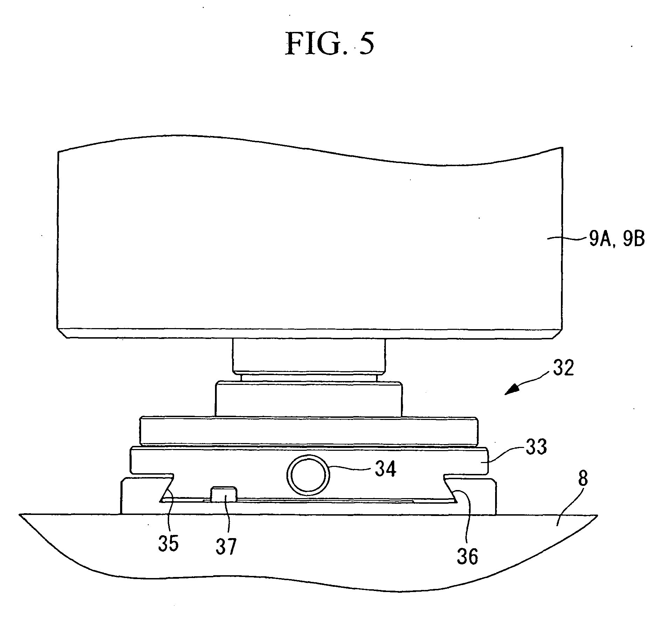

As shown in Fig. 5, the slider 33 has a dovetail 36, which is fitted in a dovetail groove 35 secured to the top plate 8 in a manner allowing it to slide and which is secured so as not to fall off. In addition, stoppers 37 against which the slider 33 abuts are provided at each stroke end of the dovetail groove 35.

The CCD cameras 9A and 9B are each secured to the slider 33 by the same type of position adjustment mechanisms 19 as that in the first embodiment. Accordingly, it is possible to align the optical axis and the focal positions using the position adjustment mechanisms 19 while the slider 33 abuts against the stoppers 37 at each stroke end.

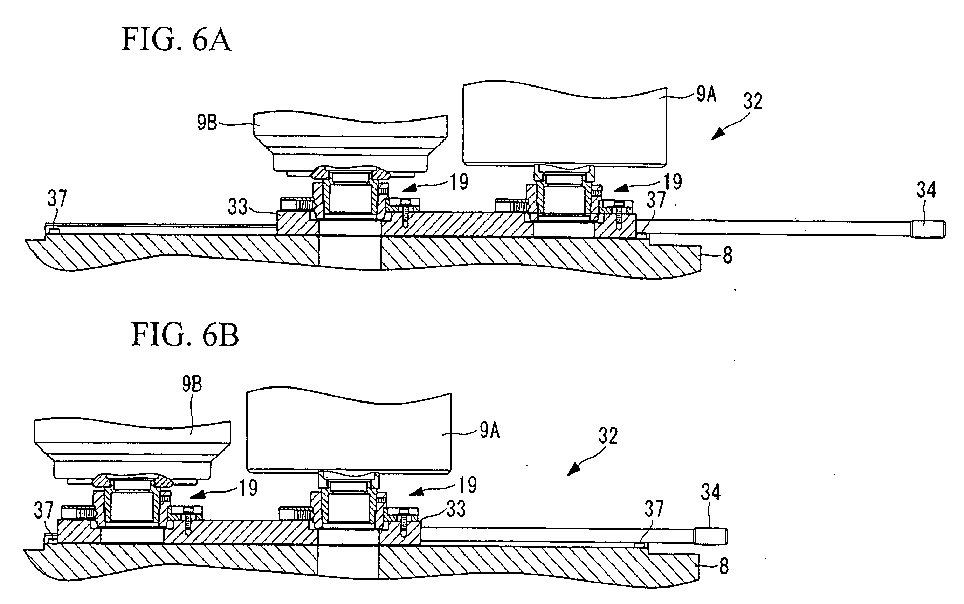

With the observation apparatus 31 according to this example, having such a configuration, it is possible to easily switch between the CCD cameras 9A and 9B used for different kinds of observation merely by moving the slider 33 to the stroke ends by operating the switching knob 34. The CCD cameras 9A and 9B are each aligned by the position adjustment mechanisms 19 in such a manner as to be aligned with the optical axis of the observation optical system 4 when the slider 33 abuts against the stoppers 37 (states shown in Figs. 6A and 6B). Accordingly, an advantage is afforded in that various kinds of observation can be accurately performed by easily changing the observation type.

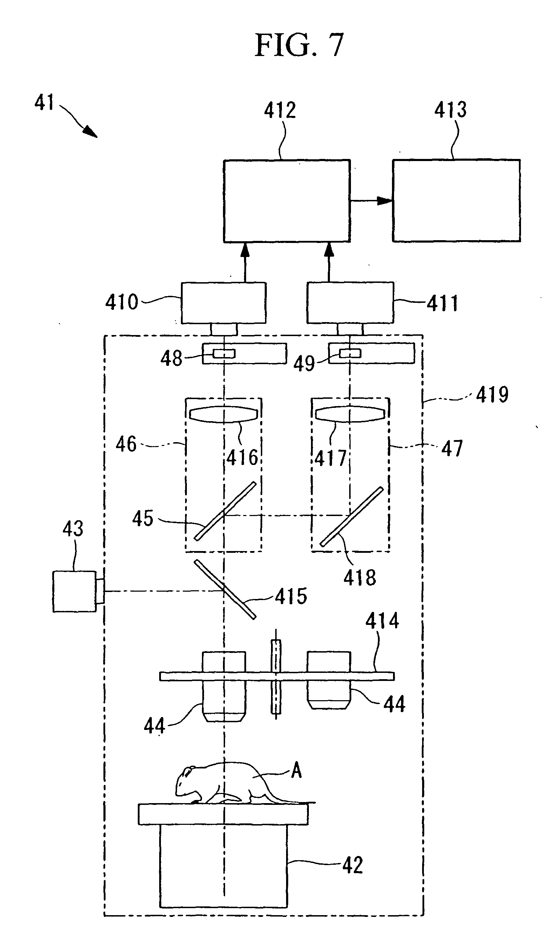

An observation apparatus 41 according to another example will be described below with reference to Fig. 7.

The observation apparatus 41 according to this example is used for observing a living organism, such as a small laboratory animal like a mouse, serving as a specimen A.

As shown in Fig. 7, the observation apparatus 41 according to this example includes a stage 42 for mounting the specimen A, such as a small laboratory animal; an illumination light source 43 for producing illumination light that irradiates the specimen A mounted on the stage 42; an objective lens 44 for irradiating the specimen A with the illumination light from the illumination light source 43 and for collecting return light from the specimen A; a first beam splitter 45 for splitting the return light collected by the objective lens 44 into two; two observation optical systems 46 and 47 for guiding the return light that is split by the first beam splitter 45; filters 48 and 49 for selectively transmitting part of the return light that is guided by each of the observation optical systems 46 and 47; and two image-acquisition units 410 and 411 for acquiring the return light transmitted from the filters 48 and 49.

Outputs from each of the image-acquisition units 410 and 411 are connected to an image processing device 412 that is connected to a monitor 413.

In the figure, reference numeral 414 is an objective lens turret that holds a plurality of the objective lenses 44 in a switchable manner, and reference numeral 419 is a black box for blocking the intrusion of external light by enclosing the observation optical systems 46 and 47, the first beam splitter 45, the objective lenses 44, the filters 48 and 49, and the stage 42 on which the specimen A is mounted.

The illumination light source 43 produces illumination light that has a relatively wide wavelength band including an excitation wavelength that can excite fluorescent material contained in the specimen A.

A second beam splitter 415 is disposed between the illumination light source 43 and the objective lens 44. The second beam splitter 415 reflects part of the illumination light from the illumination light source 43 toward the objective lens 44 and transmits part of the return light from the specimen A toward the first beam splitter 45.

The first and the second beam splitters 45 and 415 are, for example, half mirrors.

The two observation optical systems 46 and 47 include optical systems such as lenses (not shown in the drawing), as well as image-forming lenses 416 and 417 for collecting the return light. The image-forming lenses 416 and 417 each image the collected return light on image-acquisition surfaces of the image-acquisition units 410 and 411, respectively. In the figure, reference numeral 418 is a mirror.

The filters 48 and 49 are each disposed in different optical paths. Among the return light from the specimen A, the filter 48 allows transmission of the light having the same wavelength band as that of the illumination light and prevents transmission of other light; and the filter 49 prevents transmission of the light having the same wavelength band as that of the illumination light and allows transmission of other light.

The image-acquisition units 410 and 411 are both CCD cameras.

With this configuration, one image-acquisition unit 410 acquires a reflected-light image by imaging the reflected light that is the return light from the specimen A, and the other image-acquisition unit 411 acquires a fluorescence image by imaging fluorescence that is the return light from the specimen A.

The image processing device 412 superimposes the reflected-light image and the fluorescence image acquired by two image-acquisition units 410 and 411. The monitor 413 simultaneously displays the reflected-light image and the fluorescence image superimposed by the image processing device 412.

The operation of the observation apparatus 41 according to this embodiment, having such a configuration, will be described below.

In order to observe the specimen A, such as a small laboratory animal, using the observation apparatus 41 according to this embodiment, the specimen A, which has been administered or injected a fluorescent drug that is specifically accumulated in tumor tissue, such as a carcinoma, and put to sleep with anesthesia, is secured to the stage 42, and the illumination light is emitted by operating the illumination light source 43.

A portion of the illumination light emitted from the illumination light source 43 is directed toward the objective lens 44 by the second beam splitter 415 formed of the half mirror, is collected by the objective lens 44, and is radiated onto the specimen A on the stage 42.

Because the illumination light includes light having an excitation wavelength, the fluorescent drug contained in the specimen A is excited by the irradiated light of the excitation wavelength, and fluorescence having a predetermined wavelength is produced. In addition, a portion of the irradiated illumination light is reflected at the surface of the specimen A.

Accordingly, the fluorescence produced in the specimen A and the light reflected from the surface of the specimen A are collected by the objective lens 44 as the return light, and a portion thereof is transmitted through the second beam splitter 415, is directed toward the first beam splitter 45, and is split into two optical paths by the first beam splitter 45.

The observation optical systems 46 and 47 are disposed in each of the optical paths, and the respective light beams are collected by the image-forming lenses 416 and 417 provided in the observation optical systems 46 and 47. By passing through each of the filters 48 and 49, the light that is collected by the image-forming lenses 416 and 417 and that contains only a predetermined wavelength component is allowed to be transmitted, and the light having other wavelength components is not allowed to be transmitted.

Because the filter 49 provided in one optical path allows the transmission of only the fluorescence and prevents the transmission of the light having other wavelength bands, the fluorescence image is acquired by imaging the transmitted fluorescence on the image-acquisition surface of the image-acquisition unit 411. The filter 48 provided in the other optical path prevents the transmission of the fluorescence and allows the transmission of the light having other wavelength bands; therefore the reflected light transmitted therethrough is imaged on the image-acquisition surface of the image-acquisition unit 410, thus acquiring the reflected-light image.

The fluorescence image and the reflected-light image acquired by each of the image-acquisition units 410 and 411 are superimposed at the image processing device 412 and are simultaneously displayed on the monitor 413.

In this way, an observer can simultaneously observe the image in which the fluorescence image and the reflected-light image are superimposed on the monitor 413 and can easily check the position and the size of a tumor or the like displayed with the fluorescence image while observing the external view of the specimen A displayed with the reflected-light image. In particular, by superimposing the fluorescence image and the reflected-light image and displaying them, an advantage is afforded in that it is possible to simultaneously observe both images in real time.

In this case, with the observation apparatus 41 according to this example, because the return light from the specimen A is split before reaching the image-forming lenses 416 and 417, a reduction optical system having low magnification can be employed as the observation optical systems 46 and 47; therefore it is possible to separately and simultaneously observe the fluorescence image and the reflected-light image even when it is difficult to increase the space between the image-forming lenses 416 and 417 and the image-acquisition units 410 and 411.

In this example, the return light is split by the first beam splitter 45 formed of the half mirror. Instead of this, however, a dichroic mirror may be used. For example, when the wavelength range of the excitation light is from 460 nm to 490 nm, and when the wavelength of the fluorescence produced is equal to 510 nm or more, by using a dichroic mirror that splits the light at a boundary wavelength of 505 nm, the return light can be split without any loss, thus making it possible to acquire bright fluorescence and reflected-light images.

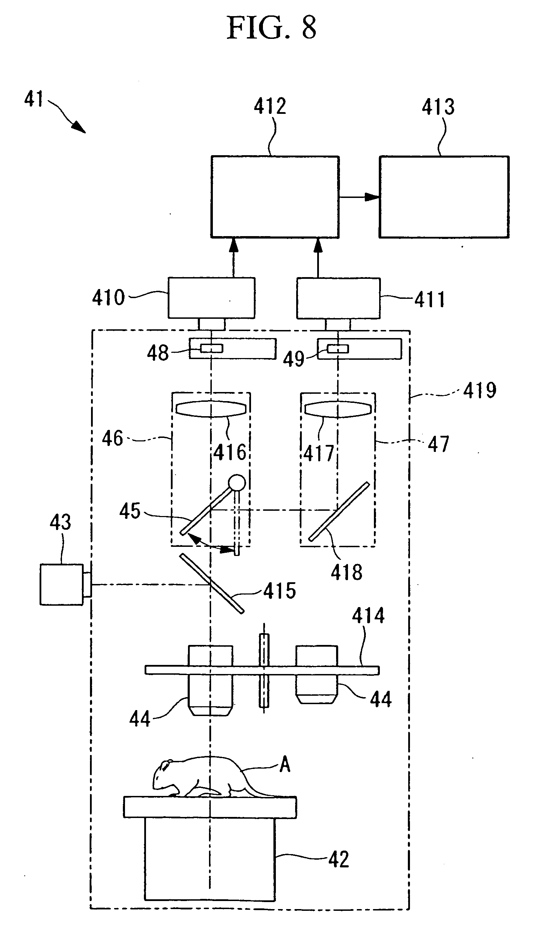

In this example, as shown in Fig. 8, the beam splitter 45 formed of a half mirror may be detachably provided in the optical path of the return light collected by the objective lens 44. By doing so, it is possible to acquire all of the return light using one of the image-acquisition units, namely the image-acquisition unit 410, thus obtaining a bright fluorescence image.

In addition, in this example, the focal lengths of the image-forming lenses 416 and 417 provided in the two observation optical systems 46 and 47 may be made to differ. By doing so, magnifications of the fluorescence image and the reflected-light image acquired by the two image-acquisition units 410 and 411 can be made to differ. In this case, the image processing device 412 may process the images in such a manner as to display the images in separate windows on the monitor 413 instead of superimposing the two images.

In addition, the image-forming lenses 416 and 417 may be movably disposed in the optical-axis direction. By doing so, focusing can be carried out with the image positions of the image-forming lenses 416 and 417 aligned with the optical-axis direction.

A turret (not shown in the drawing) for holding a plurality of the image-forming lenses 416 and 417 in a replaceable manner may be provided. By doing so, the observation magnification can be easily changed by replacing the image-forming lenses 416 and 417 by rotating the turret.

As the image-acquisition units 410 and 411, it is also possible to use units in which a color image is obtained and in which a monochrome image is obtained, respectively. This provides an advantage in that, with color images, a visible observation image can be easily recognized, whereas, with monochrome images, luminance analysis can be easily carried out.

In the above example, a case where a fluorescence image and a reflected-light image are obtained is described. However, an emitted-light image and a reflected-light image may be separately and simultaneously observed using a luminous gene such as luciferase. In addition, a fluorescence image and an emitted-light image may be separately and simultaneously observed. In this case, it is preferable to use a fluorescence image to confirm locality and to use an emitted-light image for quantitative analysis.

## Claims

1. An observation apparatus (1) comprising:
a stage (3) configured to mount a specimen (A);
an illumination light source (15) configured to emit illumination light for irradiating the specimen (A);
an objective lens (13) configured to collect return light from the specimen (A);
an image-forming lens (14) configured to image the return light collected by the objective lens (13);
an image-acquisition unit (9) configured to acquire the return light imaged by the image-forming lens (14); and
an attaching-and-detaching mechanism (10) configured to attach and detach the image-acquisition unit (9) in such a manner that the image-acquisition unit (9) is capable of being positionally aligned with an optical axis of the image-forming lens (14),
**characterized in that**
the attaching-and-detaching mechanism (10) comprises a position adjustment mechanism (19), which is configured, after the attachment of the image-acquisition unit (9), to move and secure positions of the image-acquisition unit (9) in a direction of the optical axis of the image-forming lens (14) and a direction orthogonal to the optical axis of the image-forming lens (14), wherein
the attaching-and-detaching mechanism (10) is detachable together with the image-acquisition unit (9),
the position adjustment mechanism (19) is configured to positionally align the image-acquisition unit (9) with the optical axis of the image-forming lens (14), and
the position adjustment mechanism (19) can independently move the image-acquisition unit (9) in the direction of the optical-axis of the image-forming lens (14) and in the direction orthogonal to the optical axis.

## Patentansprüche

1. Beobachtungsvorrichtung (1), aufweisend:
eine Plattform (3), die dazu eingerichtet ist, eine Probe (A) aufzunehmen;
eine Beleuchtungslichtquelle (15), die dazu eingerichtet ist, Beleuchtungslicht zum Bestrahlen der Probe (A) zu emittieren;
eine Objektivlinse (13), die dazu eingerichtet ist, von der Probe (A) zurückkehrendes Licht zu sammeln;
eine bildformende Linse (14), die dazu eingerichtet ist, das von der Objektivlinse (3) gesammelte, zurückkehrende Licht abzubilden;
eine Bilderfassungseinheit (9), die dazu eingerichtet ist, das von der bildformenden Linse (14) abgebildete, zurückkehrende Licht zu erfassen; und
einen Anbringungs- und Entfernungs-Mechanismus (10), der dazu eingerichtet ist, die Bilderfassungseinheit (9) auf eine solche Weise anzubringen und zu entfernen, dass die Bilderfassungseinheit (9) dazu geeignet ist, mit einer optischen Achse der bildformenden Linse (14) hinsichtlich der Position ausgerichtet zu sein,
**dadurch gekennzeichnet, dass**
der Anbringungs- und Entfernungs-Mechanismus (10) einen Positionsanpassungsmechanismus (19) aufweist, der dazu eingerichtet ist, nach der Anbringung der Bilderfassungseinheit (9) Positionen der Bilderfassungseinheit (9) in eine Richtung der optischen Achse der bildformenden Linse (14) und eine Richtung orthogonal zu der optischen Achse der bildformenden Linse (14) zu bewegen und zu sichern, wobei
der Anbringungs- und Entfernungs-Mechanismus (10) zusammen mit der Bilderfassungseinheit (9) entfernbar ist,
der Positionsanpassungsmechanismus (19) dazu eingerichtet ist, die Bilderfassungseinheit (9) mit der optischen Achse der bildformenden Linse (14) hinsichtlich der Position anzupassen, und
der Positionsanpassungsmechanismus (19) die Bilderfassungseinheit (9) unabhängig in die Richtung der optischen Achse der bildformenden Linse (14) und in die Richtung orthogonal zu der optischen Achse bewegen kann.

## Revendications

1. Appareil d'observation (1) comprenant:
une platine (3) configurée pour supporter un spécimen (A);
une source de lumière d'éclairement (15) configurée pour émettre une lumière d'éclairement destinée à irradier le spécimen (A);
une lentille d'objectif (13) configurée pour collecter la lumière de retour à partir du spécimen (A);
une lentille de formation d'image (14) configurée pour imager la lumière de retour collectée par la lentille d'objectif (13);
une unité d'acquisition d'image (9) configurée pour acquérir la lumière de retour imagée par la lentille de formation d'image (14); et
un mécanisme de fixation et de détachement (10) configuré pour fixer et détacher l'unité d'acquisition d'image (9) de manière à ce que l'unité d'acquisition d'image (9) soit apte à être mise en position alignée avec un axe optique de la lentille de formation d'image (14),
**caractérisé en ce que**:
le mécanisme de fixation et de détachement (10) comprend un mécanisme de réglage de position (19) qui est configuré, après la fixation de l'unité d'acquisition d'image (9), pour déplacer et maintenir des positions de l'unité d'acquisition d'image (9) dans une direction de l'axe optique de la lentille de formation d'image (14) et une direction orthogonale à l'axe optique de la lentille de formation d'image (14), dans lequel
le mécanisme de fixation et de détachement (10) peut être détaché conjointement à l'unité d'acquisition d'image (9),
le mécanisme de réglage de position (19) est configuré pour aligner la position de l'unité d'acquisition d'image (9) avec l'axe optique de la lentille de formation d'image (14), et
le mécanisme de réglage de position (19) peut déplacer de manière indépendante l'unité d'acquisition d'image (9) dans la direction de l'axe optique de la lentille de formation d'image (14) et dans la direction orthogonale à l'axe optique.
